# EUROPÄISCHE PATENTANMELDUNG

(11) **EP 3 689 408 A1**
(43) Veröffentlichungstag der Anmeldung: **05.08.2020**
(21) Anmeldenummer: 20155138.9
(22) Anmeldetag: 03.02.2020
(51) Int. Cl.: A61M 16/06

(54) **ANÄSTHESIESCHUTZVORRICHTUNG**

(30) Priorität: 04.02.2019 CH 1262019
(71) Anmelder: Hartmann-Forth, Thomas, 5522 Tägerog AG (CH)
(72) Erfinder: Hartmann-Forth, Thomas, 5522 Tägerog AG (CH)
(74) Vertreter: Schneider Feldmann AG Patent- und Markenanwälte

(57) **Zusammenfassung**

Die Erfindung betrifft eine Anästhesieschutzvorrichtung für einen Patienten, welcher vor einem operativen Eingriff appliziert wird, wobei die Anästhesieschutzvorrichtung einfacher und schneller anlegbar sein soll. Dies wird dadurch erreicht, dass die Anästhesieschutzvorrichtung von einem Anästhesiestrumpf (1) mit schlauchförmiger Gestalt, umfassend eine Textilhülle (10) aus einem Gewirk oder Gestrick mit einer Aussenfläche (100) und einer Innenfläche (101) gebildet ist und mindestens ein Schutzpolster (11) an der Textilhülle (10) befestigt angeordnet ist und mindestens ein Befestigungsmittel (13) für Schläuche und Kabel (3) auf der Aussenfläche (100) des Anästhesiestrumpfes (1) angeordnet ist.

## Beschreibung

### Technisches Gebiet

Die vorliegende Erfindung beschreibt eine Anästhesieschutzvorrichtung für einen Patienten, welcher vor einem operativen Eingriff appliziert wird.

### Stand der Technik

Vorrichtungen zur Atemwegssicherung bzw. allgemeinen Patientensicherung in der Anästhesie, sind bereits bekannt.

Zum Schutz des Kopfes, beispielsweise während einer Schilddrüsenoperation, von Patienten, werden heute meist aufwändige Wickel aus Gaze und Verbandsmaterial vor jeder Operation individuell angefertigt und dem Patienten nach der Narkoseeinleitung und Intubation angelegt. Dieser Vorgang muss vor Beginn des chirurgischen Eingriffs nach Intubation und Installation von Beatmungsschläuchen durchgeführt werden, wobei der Patient einen Kopfverband oder Kopfwickel erhält, welcher den Kopf mindestens teilweise bedeckt, sodass eine Beatmung und Überwachung des Patienten erlaubt ist. Versorgungsschläuche werden zum Kopf und in die Mund- und/oder Nasenhöhle geführt verlegt.

Vor allem der Augenbereich und der Nasenbereich müssen derart präpariert werden, dass eine ausreichende Beatmung möglich ist und der Chirurg sogar gefahrlos seine Finger, Hände oder sogar Unterarme am oder auf dem Kopf des Patienten während der Operation ablegen kann. Diese klassischen Einwegkopfverbände müssen vor der Operation an den Kopf des Patienten angepasst werden und nach Beendigung der Operation wieder entfernt werden. Gleichwohl muss die schnellstmögliche Entfernung während eines intraoperativen Notfalls gewährleistet sein. Der angepasste Kopfwickel ist jeweils eine Spezialanfertigung. Das Resultat hängt vom Können des OP-Personals ab, ist zeitaufwändig in der Anpassung und die erreichten Ergebnisse sind oft sehr unterschiedlich, da es einen grossen Herstellungsspielraum gibt.

In der WO2011019495 ist ein Anästhesieschutz gezeigt, welcher aus einer schaumartigen Lage gebildet ist, welche um den Kopf eines Patienten gelegt werden kann. Dabei sind Aussparungen in die Anästhesieschutzvorrichtung eingebracht, sodass Augen, Mund und/oder Nase von aussen zugänglich sind, der Kopf aber ansonsten abgedeckt ist. Die beschriebene Anästhesieschutzvorrichtung wird vor der Operation um den Kopf des Patienten gelegt und verschlossen, wobei Verschlussmittel, wie ein Reissverschluss oder Klebestreifen verwendet werden. Damit wird die ein- oder mehrlagige Schaumlage am Kopf lösbar befestigt. Die vor allem zu schützenden Bereiche, wie der Augenbereich, sind hier aber meist freigelassen, was nachteilig ist. Diese Bereiche müssten entsprechend mit zusätzlichen Vorrichtungen verdeckt werden, was zu Mehraufwand führt. Die Fixierung der Anästhesieschutzvorrichtung aus einer Kunststoffschaumlage ist aufwändig und erfolgt über eine Vielzahl von Verschlussmitteln, die am Rand der Kunststoffschaumlage verteilt sind.

Nachteilig an dieser Anästhesieschutzvorrichtung ist ausserdem, dass die Kunststoffschaumlage teilweise weit von den Konturen des Kopfes beabstandet bleibt. Die Kunststoffschaumlage oder Kunststofflage ist zwar flexibel, aber da die Dicke ausreichend dick gewählt werden muss, um Gesichtsbereiche gegen mechanische Einwirkungen zu schützen, wird eine röhrenförmige Hülle um den Kopf des Patienten erreicht. Folglich gibt es Bereiche der Anästhesieschutzvorrichtung, welche sehr weit von der Oberfläche des Kopfes des Patienten entfernt sind. Eine solche Anästhesieschutzvorrichtung ist nur unzureichend auf verschiedene Kopfgrössen anpassbar. Es müssen in jedem Fall für unterschiedlich gross Köpfe unterschiedlich dimensionierte Kunststoffschaumlagen vorhanden sein. Das OP-Personal ist damit sehr unflexibel mit der Anpassung unterschiedlicher Kunststoffschaumlagen an unterschiedliche Patienten.

Aufgrund des gewählten Kunststoffschaums mit nur wenigen kleinen Öffnungen wird es im Raum zwischen der Anästhesieschutzvorrichtung und der Kopfoberfläche des Patienten stickig und feuchtwarm, da die erwärmte feuchtwarme Luft nur schlecht zwischen Kopfoberfläche des Patienten und Innenfläche der Anästhesieschutzvorrichtung wegströmen kann. Hier konnte das OP-Personal bislang nur mit einer regelmässigen Belüftung reagieren.

Die beschriebenen Anästhesieschutzvorrichtungen können neben Schilddrüsenoperationen auch bei anderen operativen chirurgischen Eingriffen genutzt werden. Die beschriebenen Nachteile sind aber entsprechend auch problematisch und sollten gelöst werden.

### Darstellung der Erfindung

Die vorliegende Erfindung hat sich zur Aufgabe gestellt eine Anästhesieschutzvorrichtung zu schaffen, welche die obigen Nachteile nicht aufweist.

Die Anästhesieschutzvorrichtung soll einfach und schnell anlegbar sein und trotzdem einen ausreichenden Schutz liefern und Schläuche und Kabel während der Operation geführt halten.

Zur Lösung dieser Aufgabe wird eine Anästhesieschutzvorrichtung in Form eines Anästhesiestrumpfes mit einer Textilhülle nach Patentanspruch 1 eingeführt. Weitere vorteilhafte Abwandlungen werden von den abhängigen Patentansprüchen beansprucht.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.

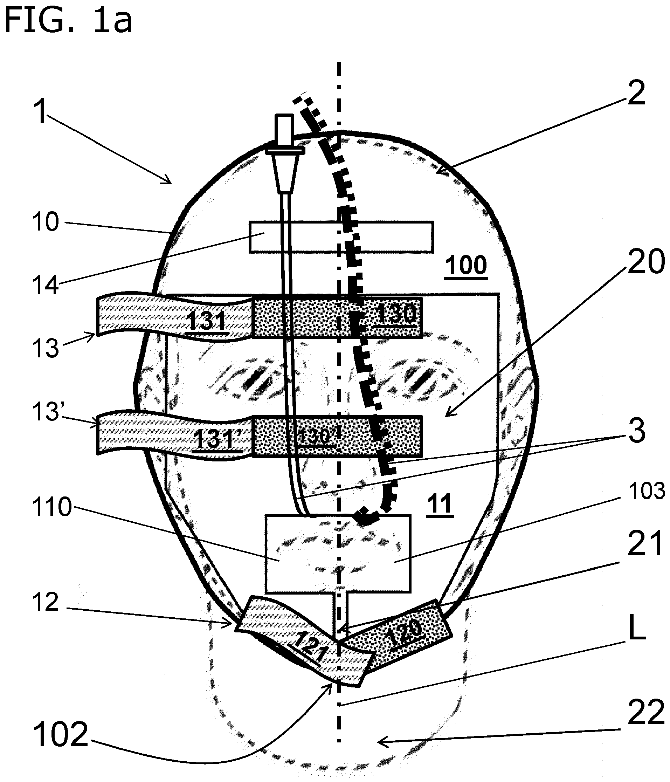

- Figur 1a: zeigt eine schematische Ansicht eines Kopfes eines Patienten mit einem angelegte Anästhesiestrumpf vor der Befestigung von Intubationsschläuchen und Kabeln vor dem operativen Eingriff.
- Figur 1a: eine schematische Schnittansicht eines Kopfes in Profilansicht mit übergezogenem Anästhesiestrumpf, wobei Hüllenverschlussmittel und mindestens ein Schutzpolster gezeigt sind.
- Figur 2a: zeigt eine schematische Frontansicht einer weiteren Ausführungsform des Anästhesiestrumpf an einem schematisch angedeuteten Kopf, während
- Figur 2b: eine schematische Schnittansicht eines Kopfes in Profilansicht mit dem Anästhesiestrumpf gemäss Figur 2a zeigt.

### Beschreibung

Zum Schutz des Patienten bei operativen chirurgischen Eingriffen wird eine Anästhesieschutzvorrichtung in Form eines Anästhesiestrumpfes 1 eingeführt. Beispielhalft wird der Anästhesiestrumpf 1 hier zum Schutz des Kopfes 2 eines Patienten während einer Schilddrüsenoperation gezeigt und seine Funktion erläutert. Derartige Anästhesiestrümpfe 1 können aber auch bei anderen chirurgischen Eingriffen verwendet werden.

Der Anästhesiestrumpf 1 wird von einer Textilhülle 10, in Form eines Gewirkes oder Gestricks, gebildet. Der Anästhesiestrumpf 1 muss eine schlauchförmige Gestalt aufweisen und elastisch genug sein, damit ein Anschmiegen der Anästhesieschutzvorrichtung 1 an die Hautoberfläche des Patienten erfolgen kann. Die schlauchförmige Gestalt muss im angelegten Zustand der Textilhülle 10 erreicht sein.

Durch Wahl einer gewirkten und/oder gestrickten Textilhülle 10 ist eine gewünschte Atmungsaktivität erreicht, sodass feuchtwarme Luft von der Haut des Patienten entweichen kann.

In der einfachsten Ausführungsform weist der Anästhesiestrumpf 1 die Textilhülle 10, ein Schutzpolster 11, ein Hüllenverschlussmittel 12 und ein Befestigungsmittel 13 auf.

Der Anästhesiestrumpf 1 bzw. die Textilhülle 10 weist eine Aussenfläche 100 und eine der Patientenhaut zugewandte Innenfläche 101 auf. Damit die Textilhülle 10 über einen Körperbereich des Patienten, hier über den Kopf 2 gezogen werden kann, muss die Textilhülle 10 mindestens eine untere Öffnung 102 aufweisen. Der Anästhesiestrumpf 1 wird in Richtung einer Längsachse L über den Kopf 2 gezogen, wobei das Gesicht 20 des Patienten und der Hinterkopf bedeckt werden. Der Anästhesiestrumpf 1 ragt bis zum Kinn 21 oder bis zum Hals 22 des Patienten und schmiegt sich aufgrund seiner elastischen Gestaltung an die Konturen des Gesichts 20 an. An der Position des mindestens einen Schutzpolsters 11 liegt der Anästhesiestrumpf 1 mit dem mindestens einen Schutzpolster 11 auf der Gesichtshaut auf.

Zur sicheren Befestigung der Textilhülle 10 wird das mindestens eine Hüllenverschlussmittel 12 eingesetzt. Mit den Hüllenverschlussmitteln 12 wird die untere Öffnung 102 der Textilhülle 10 im Bereich des Kinns 21 oder des Halses 22 lösbar verschlossen. Damit nach dem Anlegen des Anästhesiestrumpfes 1 ein späteres Verrutschen der Textilhülle 10 ausgeschlossen wird oder werden kann. Da ein atmungsaktives textiles Gewirk oder Gestrick verwendet wird, kann der Patient bzw. die Haut des Patienten selbst bei Überzug des Anästhesiestrumpfes 1 über das Gesicht immer selbstständig atmen.

Im Bereich des Mundes des Patienten ist hier in der Textilhülle eine Mundaussparung 103 ausgespart, durch welche Schläuche 3 und Kabel durchgeführt werden. Diese Mundaussparung 103 könnte erst direkt vor dem Anlegen an einen Patienten in die Textilhülle 10 eingebracht werden.

Die Textilhülle 10 könnte eine hier nicht dargestellte optionale obere Öffnung aufweisen, welche auf der, der Kinnseite gegenüberliegenden Seite angeordnet ist. Für operative Eingriffe im Bereich des Kopfes oder Halses, bietet sich aber die oben geschlossene Ausführungsform nur mit einer unteren Öffnung 102 an.

Ein weiterer optionaler Zugang zum Gesicht des Patienten, für Elektroden, Schläuche oder Kabel, ist hier durch die optionale Zusatzstrumpföffnung 14, beabstandet vom Schutzpolster 11, hier im Bereich der Stirn des Kopfes 2, ausgespart.

Im Bereich des Gesichtsfeldes des Patienten ist hier auf der Innenfläche 101 der Textilhülle 10 das mindestens eine Schutzpolster 11 angeordnet. Das Schutzpolster 11 weist zur Gesichtsoberfläche hin und kann teilweise auf der Haut des Patienten aufliegen, während es nach aussen von der Textilhülle 10 verdeckt ist. Das Schutzpolster 11 sollte so ausgebildet und angeordnet sein, dass der Bereich der Augen des Gesichts 20 des Patienten vor äusseren Einflüssen geschützt ist. Bevorzugt ist auch der Bereich der Wangen, der Nase und des Mundes mindestens teilweise verdeckt.

Um diese Abdeckung zu erreichen, kann ein zusammenhängendes einstückiges Schutzpolster 11 verwendet werden, wie hier dargestellt. Damit in den Mund und/oder der Nase des Patienten Schläuche und Kabel 3 zugeführt werden können, ist eine Mundbereichsaussparung 110 im einstückigen Schutzpolster 11 vorgesehen.

Es können aber auch mehrere Schutzpolster 11, beispielsweise in Streifenform oder komplexere Formen bildend ausgeführt auf der Innenfläche der Textilhülle 10 verteilt und dort unlösbar befestigt werden, welche dann das Schutzpolster 11 bilden. Zwischen diesen Einzelteilen oder als Ausschnitt in einem Einzelteil kann dann die Mundbereichsaussparung 110 vorgesehen sein.

Das Schutzpolster 11 sollte eine Dicke von mindestens einem Zentimeter, bevorzugt mindestens zwei Zentimetern aufweisen. Die Schutzpolster 11 können einstückig aus einem Material gefertigt sein. Schaummaterialien bieten sich als Schutzpolster 11 an, wobei diese auch von einer Textillage umhüllt, an dieser befestigt sein sollten.

Das Schutzpolster 11 oder mehrere Einzelteile, die ein Schutzpolster bilden, können an der Innenfläche der Textilhülle 101 angenäht, angeschweisst oder angeklebt sein. Entscheidend ist, dass das Schutzpolster beim Anlegen des Anästhesiestrumpfes 1 nicht verrutscht und aufgrund der Polsterwirkung das Gesicht des Patienten geschützt ist.

Auf der Aussenfläche 100 der Textilhülle 10 ist das mindestens eine Befestigungsmittel 13 angeordnet. Das mindestens eine Befestigungsmittel 13 dient dazu die Schläuche und Kabel 3, die für eine Anaesthesie bei einem operativen Eingriff benötigt werden, während der Operation vom Patienten gesichert wegzuführen. Dabei soll die örtliche Fixierung der Schläuche und Kabel 3 erreicht werden und ein Verrutschen und Verklemmen ausgeschlossen sein. Hier sind zwei Befestigungsmittel 13, 13' auf der Aussenfläche 100 der Textilhülle 10 örtlich voneinander in unterschiedlichen Höhen des Anästhesiestrumpfes 1 entlang der Längsachse L angeordnet.

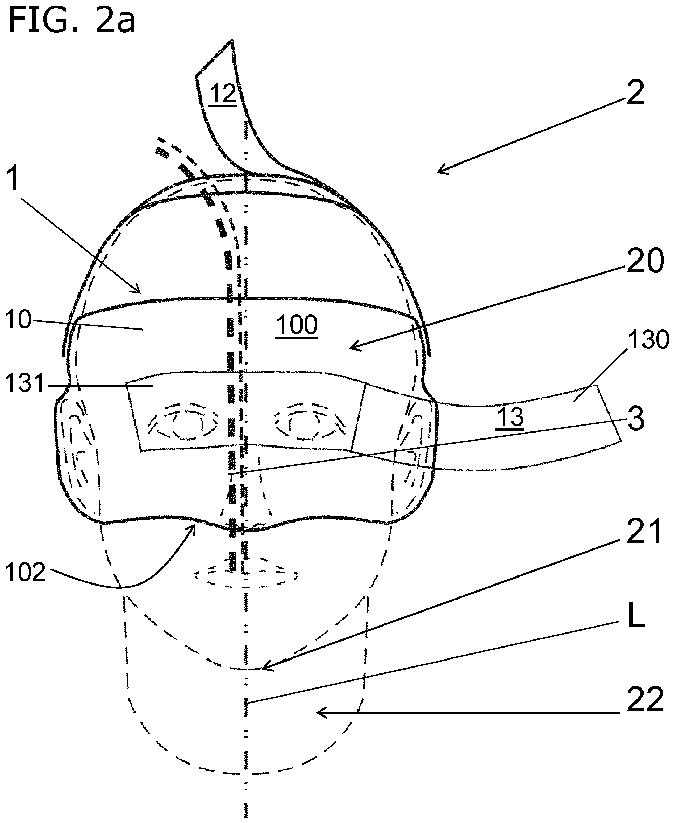

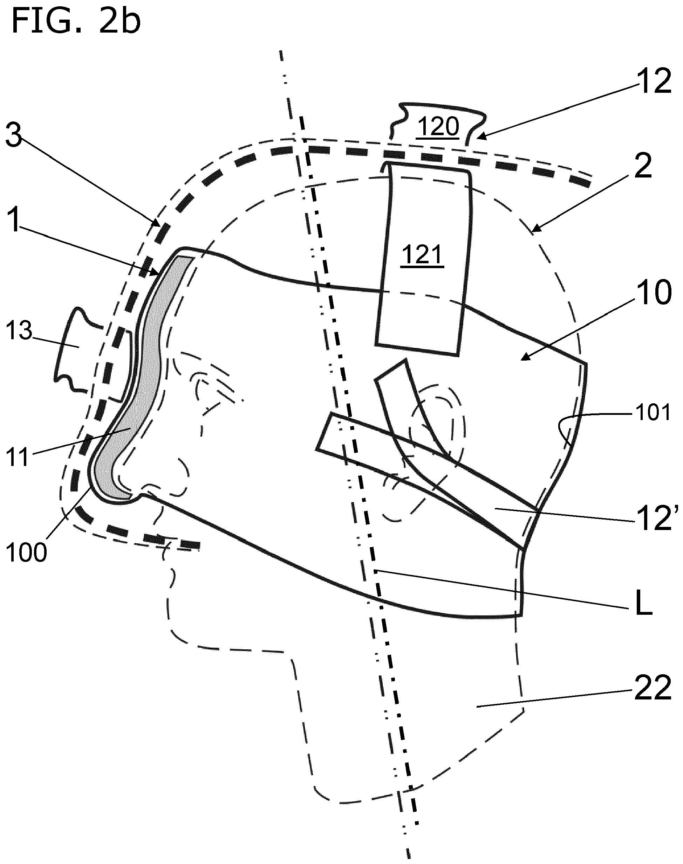

Als mindestens ein Befestigungsmittel 13 und als Hüllenverschlussmittel 12 werden bevorzugt Klettverschlüsse, umfassend ein Hakenband 120, 130 und ein Flauschband 121, 131 eingesetzt. Entweder werden die Hakenbänder 120, 130 oder die Flauschbänder 121, 131 auf der Aussenfläche 100 der Textilhülle 10 durch Annähen oder eventuell Anschweissen befestigt, während das jeweils andere Hakenband 120, 130 oder Flauschband 121, 131 schwenkbar zur Textilhülle 10 bleibt. Entsprechend können Schläuche und Kabel 3 vom Mundbereich aus der Mundaussparung 103 in der Textilhülle 10 und dem Schutzpolster 11 vom Gesicht des Patienten weggeführt werden und entlang der Aussenfläche 100 der Textilhülle 10 mit den Klettverschlüssen befestigt werden. Dabei werden die Schläuche und Kabel 3 zwischen Flauschband 121, 131 und Hakenband 120, 130 geklemmt. Dieses Einklemmen ist in Figur 2 gut erkennbar.

Die Textilhülle 10 ist schlauchartig geformt und liegt eng am Kopf 2 des Patienten, welcher gestrichelt angedeutet ist, an, während im Bereich des Gesichtsfeldes 20 das mindestens eine Schutzpolster 11 auf der Innenfläche 101 der Textilhülle 10 direkt auf der Gesichtshaut aufliegt. Die Textilhülle 10 ist damit in diesem Bereich von der Gesichtshaut beabstandet. Durch die Textilhülle 10 und das mindestens eine Schutzpolster 11 ist der Bereich der Augen und der Nase geschützt und ein Operateur kann die Finger oder eine Hand zur Fixierung gefahrlos ablegen. Auf der Aussenfläche 100 sind die Hüllenverschlussmittel 12 und die Befestigungsmittel 13, 13' angeordnet. Die Hüllenverschlussmittel 12 sorgen dafür, dass die Textilhülle 10 und der gesamte Anästhesiestrumpf 1 nicht verlierbar sind und auch nicht Verrutschen. Zwischen den Befestigungsmitteln 13, 13' sind die Schläuche und Kabel 3 sicher geführt gehalten. Die Schläuche und Kabel 3 können sich nicht lösen und auch keine Druckstellen am Patienten erzeugen.

Das Material für die Hüllenverschlussmittel 12 und/oder die Befestigungsmittel 13, 13' bzw. die Klettverschlüsse ist aus Polyamid-, Polyester-, Polyaramid- und/oder Polyolefinfasern, meist gewebt hergestellt. Bei den Hakenbändern sind die Haken während des Webens oder später eingearbeitet. Hier sind mit Klettverschluss alle möglichen Arten von Haken- und Flauschverschlüssen gemeint, welche bereits seit längerem in der Textilindustrie eingesetzt werden.

In einer leichten Abwandlung des Anästhesiestrumpfes 1, kann die Textilhülle 10 wie oben bereits beschrieben mit einer zusätzlichen oberen Öffnung versehen und damit als Band geformt sein, welches wie in Figur 2a gezeigt, um das Gesicht 20 eines Patienten gelegt werden kann. Dabei handelt es sich bei der Bandform um einen dünnen Schlauch in Richtung Längsachse L im angezogenen und befestigten Zustand. Wenn eine offene Bandform gewählt ist, weist die Textilhülle 10 zwei Enden auf, die hier im Bereich des Hinterkopfes zusammengeführt und dort befestigt sind. Die Textilhülle 10 kann aber auch als geschlossenes Band gewählt sein, wobei die Hüllenverschlussmittel 12 das Band in Richtung Kopfumfang zusammenziehen. Durch die Ausgestaltung als Band, ist keine extra Mundaussparung nötig, wobei der Mund aber frei liegen soll.

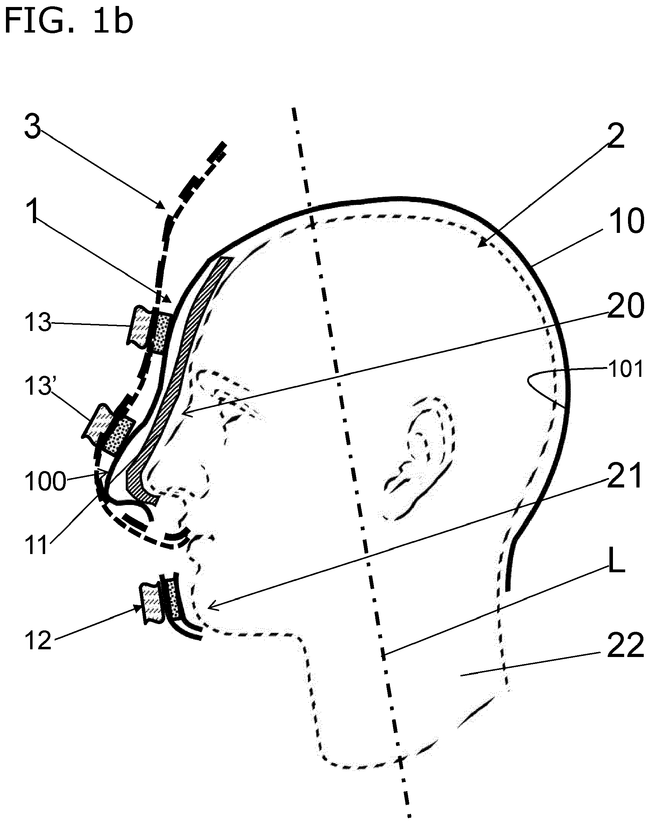

Im Bereich der Augen und der Nase ist das mindestens eine Schutzpolster 11 angeordnet, um für den Schutz zu sorgen. Zum Halten der Schläuche und Kabel 3 ist auf der Aussenfläche 100 der Textilhülle 10 mindestens ein Befestigungsmittel 13 vorgesehen. Auch hier bietet sich ein Klettverschluss mit Hakenband 130 und Flauschband 131 wieder an. Der Mund, das Kinn 21 und der Hals 22 können bei geeigneter Wahl der Höhe der Textilhülle 10 frei liegen. Um die Textilhülle 10 zusätzlich gegen ein Wegrutschen zu sichern ist ein weiteres Hüllenverschlussmittel 12, über den Kopf führend, die obere Öffnung überspannend, an der Textilhülle 10 befestigt vorgesehen. Diese Textilhülle 10 kann ansonsten alle Merkmale der Ausführungsform der Figuren 1 aufweisen. Vorteilhaft ist hier die vereinfachte Anordnung der Textilhülle 10 am Kopf 2 eines Patienten. Auch eine Befestigung an den Extremitäten ist hier ohne Weiteres möglich.

Das vereinfachte Befestigen ist in Figur 2b deutlich erkennbar. Die Hüllenverschlussmittel 12' im Bereich der Enden der bandförmigen Textilhülle 10 sind bevorzugt als Hakenband 120 und Flauschband 121 gebildet und können den Umfang der Textilhülle 10 derart verringern, dass die Textilhülle justiert bleibt, selbst bei Bewegungen des Kopfes 2. Da vor allem die Augen geschützt werden sollen, ist das Schutzpolster 11 entsprechend an der Innenfläche 101 der Textilhülle 10 befestigt, während die Befestigungsmittel 13 und die Hüllenverschlussmittel 12 an der Aussenfläche 100 der Textilhülle 10 befestigt sind.

Da die bandförmige Textilhülle 10 der Ausführungsform der Figuren 2 im angezogenen und befestigten Zustand eine Strumpfartigkeit besitzt, wird diese auch als Anästhesiestrumpf 1 definiert, kann aber auch Anästhesiehaube genannt werden.

Bevorzugt wird als Textilhülle 10 ein Gestrick, insbesondere ein Jersey oder Trikotstoff verwendet. Die Textilhülle 10 ist also ein weicher, elastischer Stoff. Das Gestrick ist aus Viskose oder Viskosemischungen, Kunstfasern im Allgemeinen, Wollmischgarnen oder Baumwolle herstellbar, welches atmungsaktiv und saugfähig ist.

Geeignete Materialien für das Garn des Anästhesiestrumpfes 1 sind bekannt und können Natur- und/oder Kunststofffasern umfassend. Bevorzugt sind die Garne aus hypoallergenen Materialien gebildet. Das Material sollte frei von Duft-, Farb- und Konservierungsstoffen sein, also frei von Stoffen mit hohem allergologischen Risiko. Das Garn der Textilhülle 10 bzw. mindestens die Innenfläche 101 der Textilhülle 10 sollte kontaktallergenfrei ausgebildet sein. Zusätzlich kann auf der Textilhülle 10 eine antibakterielle und/oder antimikrobielle Beschichtung angebracht sein kann.

Bevorzugt wird die Textilhülle 10 aus einem Flachstrick oder Schlauchware konfektioniert, wobei die Schutzpolster 11 Einlagen aus PE-VIies oder PU-Schaum sind. Die Hüllenverschlussmittel 12, 12' und/oder die Befestigungsmittel 13, 13' sind aus Micro-Klett und auf die Textilhülle 10 aufgenäht, wobei auch sogenannter elastischer Klett verwendet werden kann.

### Verwendung sämtlicher Ausführungsformen

Nach erfolgter Narkoseeinleitung und Intubation, kann ein Augenschutz angelegt werden, bevor der Anästhesiestrumpf 1 über den Kopf 2 gezogen wird. Am Kinn 21 werden die Klettverschlüsse 12 individuell je nach Kopfumfang abgestimmt geschlossen, sodass der Anästhesiestrumpf 1 mit dem Kinn 21 abschliesst und nicht mehr verrutscht. Nun können alle Schläuche und Kabel 3 mittels der zwei Klettverschlüsse 13, 13' über dem Schutzpolster 11 sicher fixiert werden, wobei vor allem die Augen geschützt werden. Der Zeitaufwand zum Anlegen des Anästhesiestrumpfes 1 beträgt etwa 60 Sekunden.

Der hier beschriebene Anästhesiestrumpf 1 kann, neben Schilddrüsenoperationen auch für andere operative Eingriffe am Kopf 2, Hals oder auch im Bereich der Extremitäten eingesetzt werden, wobei gegebenenfalls die untere Öffnung 102 und die obere Öffnung an der Textilhülle 10 ausgebildet sein muss. Der Bereich mit dem Schutzpolster 11 verdeckt dann nicht den Bereich der Augen oder das Gesichtsfeld, sondern andere schützbare Körperbereiche des Patienten.

### Bezugszeichenliste

1 Anästhesiestrumpf/Anästhesieschutzvorrichtung
10 Textilhülle
   100 Aussenfläche
   101 Innenfläche der Textilhülle
   102 untere Öffnung
   103 Mundaussparung
   optionale obere Öffnung
L Längsachse des Anästhesiestrumpfs
11 Schutzpolster
   110 Mundbereichsaussparung
12 Hüllenverschlussmittel
   120 Hakenband
   121 Flauschband
13, 13' Befestigungsmittel für Schläuche und Kabel
   130 Hakenband
   131 Flauschband
14 Zusatzstrumpföffnung
2 Kopf eines Patienten
20 Gesicht
21 Kinn
22 Hals
3 Schläuche und Kabel

## Patentansprüche

1. Anästhesieschutzvorrichtung für einen Patienten, welche vor einem operativen Eingriff appliziert wird,
**dadurch gekennzeichnet, dass**
die Anästhesieschutzvorrichtung von einem Anästhesiestrumpf (1) mit im angezogenen Zustand schlauchförmiger Gestalt, umfassend eine Textilhülle (10) aus einem Gewirk oder Gestrick mit einer Aussenfläche (100), einer Innenfläche (101) und mindestens einer unteren Öffnung (102) gebildet ist, mindestens ein Schutzpolster (11) an der Textilhülle (10) befestigt angeordnet ist und
mindestens ein Befestigungsmittel (13) für Schläuche und Kabel (3) auf der Aussenfläche (100) des Anästhesiestrumpfes (1) angeordnet ist.

2. Anästhesieschutzvorrichtung nach Anspruch 1, wobei das mindestens eine Schutzpolster (11) dem Innenraum des Anästhesiestrumpfes (1) zugewandt auf der Innenfläche (101) befestigt angeordnet ist.

3. Anästhesieschutzvorrichtung nach einem der vorhergehenden Ansprüche, wobei das mindestens eine Befestigungsmittel (13) auf der Aussenfläche (100) angenäht oder angeklebt ist.

4. Anästhesieschutzvorrichtung nach einem der vorhergehenden Ansprüche, wobei an der mindestens einen unteren Öffnung (102) des Anästhesiestrumpfes (1) Hüllenverschlussmittel (12) angeordnet sind, bevorzugt in Form eines Klettverschlusses, umfassend ein Hakenband (120) und ein Flauschband (121).

5. Anästhesieschutzvorrichtung nach einem der vorhergehenden Ansprüche, wobei die Textilhülle (10) zusätzlich eine obere Öffnung aufweist.

6. Anästhesieschutzvorrichtung nach Anspruch 5, wobei die Textilhülle (10) mit zweiten Hüllenverschlussmitteln (12) die obere Öffnung überspannend verschliessbar ist.

7. Anästhesieschutzvorrichtung nach einem der Ansprüche 5 oder 6, wobei an den Enden der bandförmigen Textilhülle (10) Hüllenverschlussmittel (12), in Form eines Klettverschlusses, umfassend ein Hakenband (120) und ein Flauschband (121), angeordnet sind, welche den Umfang der Textilhülle (10) fixieren.

8. Anästhesieschutzvorrichtung nach einem der vorhergehenden Ansprüche, wobei die Textilhülle (10) als Gestrick ausgestaltet ist und insbesondere ein Jersey oder Trikotstoff ist.

9. Anästhesieschutzvorrichtung nach einem der vorhergehenden Ansprüche, wobei die Textilhülle (10) mit Mundaussparung (103) und mit einem einstückig oder mehrstückig ausgestalteten Schutzpolster (11) mit einer Mundbereichsaussparung (110) ausgebildet ist.

10. Anästhesieschutzvorrichtung nach einem der vorhergehenden Ansprüche, wobei das Garn der Textilhülle (10) aus Viskose, einer Viskosemischung, Kunstfasern, einem Wollmischgarn oder Baumwolle gebildet ist.

11. Anästhesieschutzvorrichtung nach einem der vorhergehenden Ansprüche, wobei das Garn der Textilhülle (10) bzw. mindestens die Innenfläche (101) der Textilhülle (10) kontaktallergenfrei ausgebildet ist und/oder antibakteriell und/oder antimikrobiell beschichtet ist.

12. Anästhesieschutzvorrichtung nach einem der vorhergehenden Ansprüche, wobei an der Aussenfläche (100) der Textilhülle (10) zweite Befestigungsmittel (13') getrennt und örtlich beabstandet von den ersten Befestigungsmitteln (13) angeordnet, bevorzugt in Form eines Klettverschlusses, umfassend ein Hakenband (130) und ein Flauschband (131) befestigt sind.

13. Anästhesieschutzvorrichtung nach einem der vorhergehenden Ansprüche, wobei das mindestens eine Schutzpolster (11) eine Dicke von mindestens einem Zentimeter, bevorzugt mindestens zwei Zentimetern aufweist.
